# EUROPEAN PATENT SPECIFICATION

(11) **EP 3 375 884 B1**
(45) Date of publication and mention of the grant of the patent: **06.05.2020**
(21) Application number: 17161104.9
(22) Date of filing: 15.03.2017
(51) Int. Cl.: C12P 21/00, C12N 1/14, C12N 1/22

(54) **PROCESS FOR PRODUCING PROTEINS UNDER INDUCING CONDITIONS**
VERFAHREN ZUR HERSTELLUNG VON PROTEINEN UNTER INDUZIERENDEN BEDINGUNGEN
PROCÉDÉ DE PRODUCTION DE PROTÉINES DANS DES CONDITIONS INDUCTRICES

(43) Date of publication of application: 19.09.2018
(73) Proprietor: Clariant International Ltd, 4132 Muttenz (CH)
(72) Inventor: ZAVREL, Michael, 82140 Olching (DE); SCHAEFER, Rudolf, 82152 Planegg (DE); BARTUCH, Joerg, 82131 Gauting (DE); VERHUELSDONK, Marcus, 82110 Germering (DE); MAX, Sebastian, 82131 Gauting (DE)
(74) Representative: Graser, Konstanze

(56) References cited:
- EP-A2- 0 121 397
- WO-A1-2004/035070
- WO-A1-2014/110223
- ALLEN, A.L. & MORTENSEN R.E.: "Production of Cellulase from Trichoderma reesei in Fed-Batch Fermentation from Soluble Carbon Sources", BIOTECHNOLOGY AND BIOENGINEERING, vol. 23, no. 11, November 1981 (1981-11), pages 2641-2646, XP008069337,

## Description

The present invention relates to a high-performance method for the fermentative production of proteins involving the in-situ generation of an inducer substance.

Proteins are used in various applications, such as pharmaceuticals, food, feed or home care and laundry products. Production i.e. expression of the desired proteins is often carried out by fermentation processes involving yeasts, bacteria or fungi capable of expression of the desired protein. In order to yield a maximum amount of protein, the organisms are often genetically modified.

A further mechanism to increase yield is the addition of supplemental substances and/or additional substrate during fermentation, for example the feeding of so called "inducer substances" or "inducers". "Inducers" are compounds, which further increase expression rates of the desired protein by acting upon microorganisms via molecular mechanisms in a way that leads to an increased production of a target molecule.

Within the state of the art, various inducer substances such as Sophorose ((2S,3R,4S,5S,6R)-2-(hydroxymethyl)-6-[(2S,3R,4S,5S,6R)-2,4,5-trihydroxy-6-(hydroxymethyl)oxan-3-yl]oxyoxane-3,4,5-triol (alpha-Sophorose) or 2-O-beta-D-Glucopyranosyl-alpha-D-glucose; CAS Number 534-46-3) are known which overcome the problem of a likely contamination. Huang T.T. and Wages J.M. (New-to-nature sophorose analog: a potent inducer for gene expression in Trichoderma reesei; Enzyme and Microbial Technology; Volume 85, April 2016, Pages 44-50) describe the inductive effect of sophorose on gene expression in *Trichoderma reesei.* However, sophorose is expensive due to elaborate production processes and would easily increase productions costs by about 300€ per liter of fermented substrate.

Another, cheaper state of the art approach to increase protein yield is the feeding of solid substrates during fermentation, which might also contribute an inductive effect.

A problem of adding further substrate or other solids is, however, a likely contamination of the fermentation batch. External sterilization and thus sterile feeding of such substances is not feasible as it is complex and costly.

Therefore, the aim of the inventors of the present invention was to provide a method, which is neither expensive due to the incorporation of costly inducer substances nor due to elaborate process designs to avoid contamination but still guarantees high yields of the desired protein.

The inventors have now surprisingly found a method involving the in-situ generation of inducers, which provides these advantages. Moreover, the method even reduces further costs as substantially smaller amounts of alkaline or acid are needed for pH adjustment during fermentation as a higher percentage of the supplied carbon source is utilized for the production of the target enzyme and consequently less by-products are produced, which require the addition of base or acid to prevent a shift of the pH.

Further, compared to methods including the feeding of hydrolysate of e.g. cellulosic origin, the amount of enzyme needed is substantially reduced due to the continuous production and presence of intermediate substances: Many intermediate products generated during in-situ cellulose-hydrolysis boost the inductive effect. In case the cellulose hydrolysation is carried out externally in a separate step and vessel, these compounds are no longer present in the final hydrolysate and would have to be compensated by a higher dosage of enzyme.

In a first aspect, the present invention relates to a method for the production of proteins comprising the following steps:
(a) providing a fermentation medium comprising from 0.01 to 30 wt.-% cellulose;
(b) degermination of the fermentation medium;
(c) adding at least one filamentous fungus cell to the medium;
(d) adding from 0.01 to 35 wt.-% of glucose and/or saccharose over a period of at least 1 hour;
(e) adding from 0.01 to 10000 FPU cellulase per kg fermentation medium to the medium,
wherein the at least one filamentous fungus cell is a recombinant cell and
wherein the at least one filamentous fungus cell is a cell wherein the expression level of one or more cellulases has been reduced and/or one or more cellulase encoding genes have been knocked out.

Within the present invention, the term "protein" is to be understood as any protein known to a person skilled in the art as suitable for the inventive method. Preferably, the protein is selected from enzymes, wherein hydrolases are particularly preferred. Most preferably, the enzyme is selected from enzymes with main- or side activity of hydrolases such as peptidases and esterases, phytases, lyases and oxidases.

Particularly preferred are enzymes belonging to EC 3, even more preferred esterases as contained in class 3.1 and glycosylases as contained in class 3.2 and most preferred phosphoric monoester hydrolases (EC number 3.1.3.)and glucoamylases (EC number 3.2.1.3).

Within the present invention, the term "fermentation medium" is to be understood as comprising any medium known to a person skilled in the art as suitable for the inventive method. The fermentation medium preferably contains cellulose and/or glucose as carbon source. An example for a preferred growth medium is Mandels-Andreotti medium.

Within the present invention, the fermentation medium comprises form 0.01 to 30 wt.-% cellulose wherein in this context "wt.-%" pertains to the total weight of the fermentation medium, filamentous fungus cell(s), cellulase(s) and glucose and/or saccharose added until the end of the process. Within a preferred embodiment of the inventive method, the fermentation medium comprises from 0.05 to 20 wt.-% cellulose, further preferred from 0.1 to 15 wt.-% cellulose, particularly preferred from 0.25 to 10 wt.-% cellulose and most preferred from 0.5 to 7.5 wt.-% cellulose. The cellulose may be selected from any cellulose known to a person skilled in the art as suitable for the inventive method. A suitable cellulose is for example any cellulose originating from organic biomass such as but not limited to celluloses from wood, cereal straw and/or husks, bagasse, oat hulls, switch grass, cellulose, raw paper pulp (obtained from pulp and paper production) molasses and mixtures thereof. A suitable commercially available product is Arbocel® or AlbaFiber® in different qualities (preferably containing of from 50.0 wt.-% to 99.9 wt.-% cellulose, further preferred of from 65 to 99.0 wt.-%, particularly preferred of from 70.0 to 95.0 wt.-% cellulose; also preferred is cellulose in crystalline form). Preferred particle sizes of the cellulose compound are selected from 10 µm to 10 000 µm, further preferred of from 15 µm to 5000 µm, particularly preferred of from 20 to 1000 µm and most preferred of from 25 to 500 µm.

Within a preferred embodiment, the inventive method further comprises step (f): adding from 0.1 to 25 wt.-% cellulose, preferably from 0.5 to 20 wt.-% cellulose and most preferred from 1 to 15 wt.-% (wherein the wt.-% are to be determined as defined above) wherein the cellulose is added to the fermentation medium in regular quantities or in a continuous fashion and wherein step (f) is carried out after, concurrently with or during step (c), (d) or (e). "Regular quantities" are to be understood as portions of equal weight added at equal time intervals during the total run of the process. Within another embodiment of the inventive method, the fermentation medium comprises 5 to 30 wt.-%, preferably 10 to 15 wt.-% of cellulose and from 0.5 to 20 wt.-% cellulose are added during step (f) of the inventive method.

It is thereby particularly preferred to select the particle size from a range of from 15 to 500 µm when adding of from 0.1 to 25 wt.-%, preferably a range from 20 to 1000 µm when adding from 0.5 to 20 wt.-%, which guarantees a further improvement of the inductive effect while maintaining a pumpable slurry.

Within the present invention, the term "continuous fashion" or "continuously" is to be understood as a constant addition or performance of the respective step without any interruption or time-interval without addition of the respective substance or compound.

Within step (b) of the inventive method, the fermentation medium is degerminated.

The degermination can be carried out by any method known to a person skilled in the art as suitable for the inventive process. Degermination is preferably carried out by heating the fermentation medium to a temperature of at least 60 °C, preferably at least 80 °C and most preferred of at least 90 °C, wherein selection of the temperature from the range of from 60 to 150°C, preferably from 80 to 120°C is particularly preferred. Suitable and preferred methods for degermination are pasteurization, short-term heating and autoclaving. Degermination is preferably carried out for a time of from 1s to 10 hours, preferably from 10s to 5 hours and most preferred of from 15s to 1 hour. Preferred are degerminations of from 10 to 59 s at temperatures of from 122 to 150 °C in case of e.g. a continuous-flow heater or from 1 minute to 20 minutes at temperatures of from 60 to 121 °C e.g. in case of in-situ heating of the batch within the reactor. All method can either be carried out in-situ or by implementing an external, continuous-flow heater.

Within step (c) of the inventive method, at least one filamentous fungus cell is added to the fermentation medium. It is thereby preferred that the filamentous fungus is added in a quantity of from 10² to 10¹⁰ cells, preferably in a quantity of from 10³ to 10⁸ cells and most preferred in a quantity of from 10⁴ to 10⁷ cells per g of fermentation medium.

Within the present invention the term "filamentous fungus cell" is to be understood as any cell from any filamentous fungus existing in nature and/or known to a person skilled in the art. The term also comprises any filamentous fungus cell either of natural origin or wild type or genetically modified. Within a preferred embodiment the filamentous fungus cell is selected from the group consisting of *Acremonium, Aspergillus, Chaetomium, Fusarium, Humicola, Irpex, Magnaporte, Myceliophthora, Neurospora, Penicillium, Rhizopus, Talaromyces, Trichoderma* and *Trametes,* wherein *Trichoderma* and *Aspergillus* are particularly preferred, most preferred is *Trichoderma reesei.*

The at least one filamentous fungus cell is a recombinant cell. A "recombinant cell" refers to any genetically modified cell whose genetic material has been altered using genetic engineering technique. The term "genetic engineering technique" pertains to a set of technologies used to change the genetic makeup of cells, including the transfer of genes within and across species.

The at least one filamentous fungus cell is a cell wherein the expression level of one or more cellulases has been reduced and/or one or more cellulase encoding genes have been knocked out.

The "adding" according to step c) can be carried out by any method known to a person skilled in the art as suitable for the inventive purpose.

Within step (d) of the present invention 0.01 to 35 wt.-%, preferably from 0.5 to 30 wt.-%, further preferred from 1 to 25 wt.-% and most preferred of from 5 to 20 wt.-% of glucose and/or saccharose is added over a period of at least 1 hour, preferably at least 12 hours, further preferred at least 36 hours, particularly preferred at least 72 hours and most preferred at least 100 hours, wherein a period of from 5 to 500 hours is also preferred, particularly preferred of from 24 hours to 240 hours and most preferred of from 36 to 144 hours. In this context "wt.-%" pertains to the total weight of the fermentation medium, filamentous fungus cell(s), cellulase(s) and glucose and/or saccharose added until the end of the process.

Glucose is particularly preferred.

Within a preferred embodiment of the present invention step (d) is carried out in a continuous fashion. The addition is preferably performed with addition rates increasing throughout the duration of the addition. The ratio of addition rates at the end of the addition to addition rates at the beginning of the addition are preferably selected from the range of from 1.1 to 20, further preferably from 1.2 to 10 and most preferred from 1.5 to 3.

Within step (e) of the inventive method from 0.01 to 10 000 FPU (filter paper unit) cellulase per kg fermentation medium, preferably from 0.05 to 5000 FPU, further preferred from 0.1 to 3570 FPU, also preferred from 10 to 2500 FPU, particularly preferred from 15 to 1000 FPU and most preferred from 30 to 600 FPU are added to the fermentation medium. Within a preferred embodiment the 0.01 to 10 000 FPU cellulase per kg fermentation medium are added in from 1 to 500 portions, preferably from 5 to 400, further preferred from 10 to 100, also preferred from 15 to 70, particularly preferred from 17 to 50 and most preferred of from 20 to 45 portions or in a continuous fashion.

Within the present invention, the term "cellulase" is to be understood as referring to any enzyme catalyzing cellulolysis, which is the decomposition of cellulose and related polysaccharides. Within the present invention, the term "cellulase" also refers to any naturally occurring mixture or complex of such enzymes, which act serially or synergistically to decompose cellulosic material. The cellulase of the present invention may be of fungal, bacterial or protozoal origin. The term "cellulases" refers in particular to any enzyme capable of breaking down cellulose into monosaccharides such as beta-glucose, or shorter polysaccharides and oligosaccharides.

Preferred are exo- and endocellulases (i.e. Cellobiohydrolase (CBH) I, II, endoglucanase (EG) I-IV, beta-Glucosidase (BGL)). Within a preferred embodiment the cellulase has one or more activities selected from the group consisting of: Cellobiohydrolase type I or type II (CBH I or CBH II), endoglucanase type I, II, III or IV (EGI, EGII, EGIII, EGIV), beta-glucosidase (BGL).

Within another preferred embodiment, steps (c) to (e) of the inventive method are carried out concurrently or in a consecutive fashion, wherein consecutive fashion is preferred. It is thereby particularly preferred that step (d) is carried out in a continuous fashion and step (e) is carried out in that regular quantities of the cellulase are added. This embodiment is particularly preferred as by individual dosing of cellulase and saccharide, fermentation conditions can be ideally adapted to the specific fungus and substrate to guarantee the highest possible yield of the target protein.

Within another preferred embodiment of the inventive method, the pH is kept at a constant level during the whole run of the process by adding base and/or acid, wherein the base is preferably selected from ammonia, sodium hydroxide, potassium hydroxide and the acid is preferably selected from sulfuric acid, phosphoric acid and acetic acid.

Within another preferred embodiment of the inventive method, from 0.01 to 10 wt.-%, preferably from 0.5 to 5 wt.-% of an aqueous solution of ammonia (12.5 wt.-%) or the equivalent molar amount as gaseous ammonia is added.

Within another preferred embodiment of the inventive method at least steps (c) and (d) are carried out at a temperature of from 10 to 45 °C, preferably of from 20 to 35 °C and/or at a pH of from 2.5 to 9.5, preferably of from 3.5 to 8.5 and/or for a time period of from 1 minute to 20 days, preferably of from 1 day to 10 days and/or with a gassing rate of from 0.2 to 3 vvm, preferably of from 0.75 to 1.5 and/or with a a mechanical power input of from 0.01 to 20 kW/m³, preferably of from 0.1 to 10 kW/m³. It is further preferred to select a head space pressure of the fermentation medium of from 0 to 5 bar, preferably of from 0.5 to 3 bar. The "mechanical power input" is preferably generated by a stirrer of a stirred tank reactor but may also be generated by a mixer or an ultra-turrax®. Alternatively, the mechanical power input is generated by pumping the fermentation medium. This can be achieved for example by pumping the fermentation medium from the fermenter bottom through a bypass to the top of the fermenter.

In the following particularly preferred embodiments of the inventive process are described.

### Particularly preferred embodiment 1

Particularly preferred is a method for the production of proteins comprising the following steps:
(a) providing a fermentation medium comprising from 0.01 to 30 wt.-% cellulose;
(b) degermination of the fermentation medium;
(c) adding at least one filamentous fungus cell selected from *Trichoderma* or *Aspergillus* species to the medium;
(d) adding from 0.01 to 35 wt.-% of glucose and/or saccharose over a period of from 24 to 240 hours;
(e) adding from 0.01 to 10000 FPU cellulase per kg fermentation medium to the medium,
wherein the at least one filamentous fungus cell is a recombinant cell and
wherein the at least one filamentous fungus cell is a cell wherein the expression level of one or more cellulases has been reduced and/or one or more cellulase encoding genes have been knocked out.

### Particularly preferred embodiment 2

Particularly preferred is a method as defined in preferred embodiment 1, wherein at least steps (c) to (e) are carried out concurrently or in a consecutive fashion.

### Particularly preferred embodiment 3

Particularly preferred is a method as defined in preferred embodiment 2, wherein step (d) is carried out in a continuous fashion.

### Particularly preferred embodiment 4

Particularly preferred is a method as defined in preferred embodiment 1, 2 or 3, wherein the at least one filamentous fungus cell is a recombinant *Trichoderma reesei* cell wherein the expression level of one or more cellulases has been reduced and/or one or more cellulase encoding genes have been knocked out and wherein the protein is selected from enzymes from EC class 3.

### Particularly preferred embodiment 5

Particularly preferred is a method as defined in any of preferred embodiment 1 to 4, wherein the 0.01 to 10000 FPU cellulase per kg fermentation medium are added in a continuous fashion.

### Particularly preferred embodiment 6

Particularly preferred is a method for the production of proteins comprising the following steps:
(a) providing a fermentation medium comprising from 0.01 to 30 wt.-% cellulose;
(b) degermination of the fermentation medium;
(c) adding at least one filamentous fungus cell selected from *Trichoderma* species, preferably *Trichoderma reesei,* to the medium;
(d) adding from 0.01 to 5 wt.-% of glucose and/or saccharose preferably glucose, over a period of from 24 to 240 hours;
(e) adding from 0.01 to 10000 FPU cellulase per kg fermentation medium to the medium;
wherein at least steps (c) and (d) are carried out at a temperature of from 10 to 45 °C, preferably from 20 to 35 °C, at a pH of from 4.5 to 5.5 and for a time period of from 5 hours to 500 hours;
and
wherein at least steps (c) to (e) are carried out concurrently or in a consecutive fashion; and
wherein step (d) is carried out in a continuous fashion.

### Particularly preferred embodiment 7

Particularly preferred is a method as defined in preferred embodiment 6, wherein at least steps (c) and (d) are carried out with a gassing rate of from 0.2 to 3 vvm and/or at a pressure of from 1 bar to 3 bar and/or with a with a a mechanical power input of from 0.01 to 20 kW/m³.

### Particularly preferred embodiment 8

Particularly preferred is a method as defined in preferred embodiment 6 or 7, wherein the at least one filamentous fungus cell is a recombinant *Trichoderma reesei* cell, wherein the expression level of one or more cellulases has been reduced and/or one or more cellulase encoding genes have been knocked out and wherein the protein is selected from enzymes from EC class 3.

### Examples

The present invention is now described by the following examples and figures. The examples and figures are for illustrative purposes only.

### List of Figures

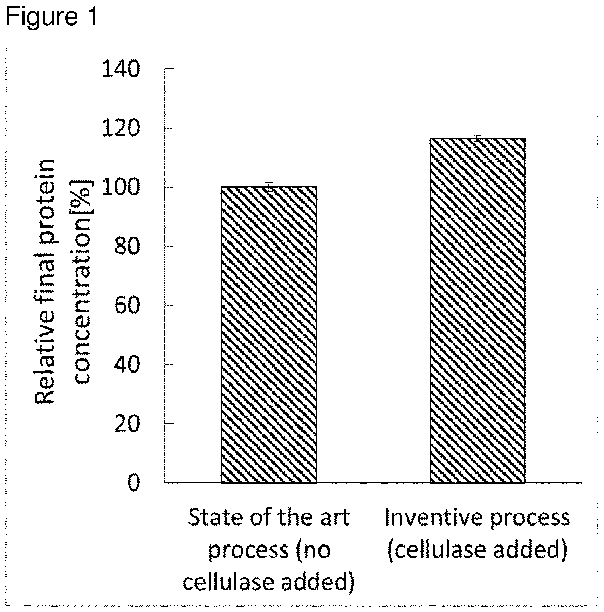

- Fig. 1: shows the relative increase in the final protein concentration of the fermentation carried out without the addition of cellulase (comparative example; left column) and with the addition of cellulase (example 1; right column) when carrying out the process of the present invention as described in example 1.

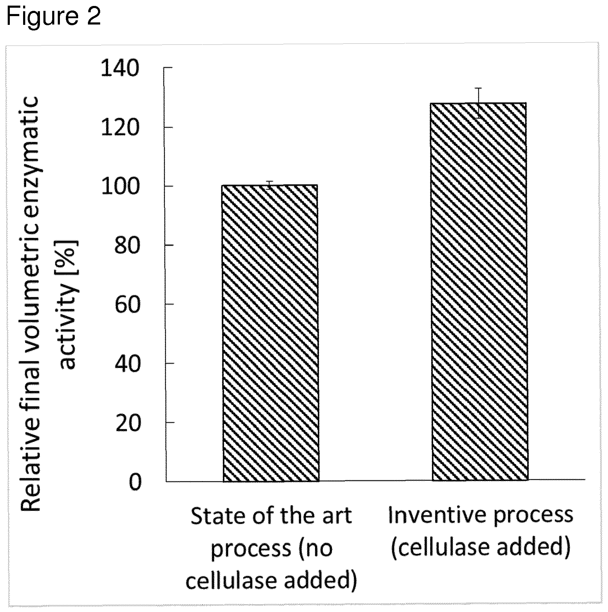

- Fig. 2: shows the relative increase in the final volumetric (units U per volume ml) enzymatic activity of the fermentation carried out without the addition of cellulase (comparative example; left column) and with the addition of cellulase (example 1; right column) when carrying out the process of the present invention as described in example 1.

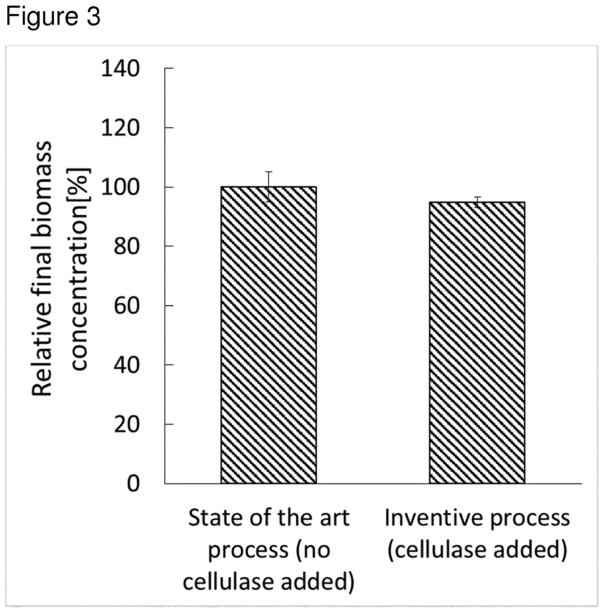

- Fig. 3: shows the relative decrease in the final biomass concentration of the fermentation carried out without the addition of cellulase (comparative example; left column) and with the addition of cellulase (example 1; right column) when carrying out the process of the present invention as defined in example 1.

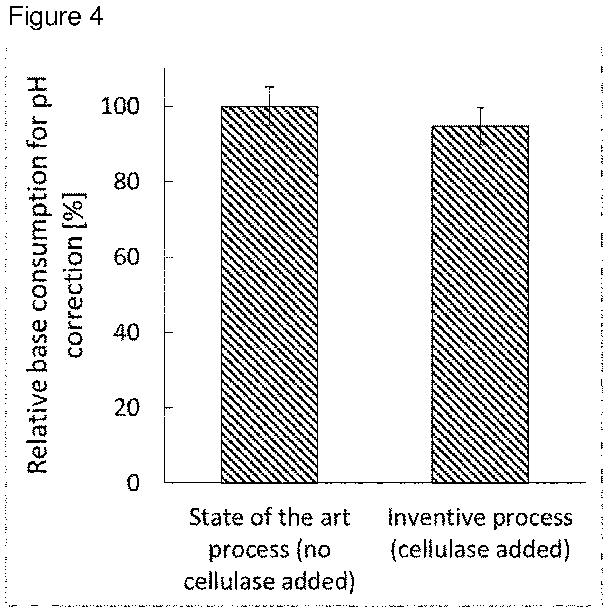

- Fig. 4: shows the relative decrease in the base (12.5 % aqueous ammonia solution) consumption for pH correction during the fermentation carried out without the addition of cellulase (comparative example; left column) and with the addition of cellulase (example 1; right column) when carrying out the process of the present invention as defined in example 1.

### Example 1:

The weight-percentages (wt.-%) in this example refer to the weight of a component relative to the final weight of the broth in the bioreactor, unless specified differently.

Fermentations were carried out in a stirred tank bioreactor system (DASGIP® Bioreactor SR0700ODLS/Bioblock, Eppendorf, Germany) in a medium containing 0.7 wt.-% glucose as the main carbon source, 0.5 wt.-% soymeal as additional carbon and nitrogen source, 1 wt.-% cellulose (Arbocel®) and additional salts and minerals (0.3 wt.-% (NH₄)₂SO₄, 0.2 wt.-% KH₂PO₄, 0.03 wt.-% CaSO₄, 0.02 wt.-% MgCl₂, 0.002 wt.-% FeSO₄ x 7 H₂0, 0.0001 wt.-% MnSO₄ x H₂O, 0.0001 wt.-%,ZnSO₄ x 7 H₂0). Both the state of the art process and the inventive process were conducted at 30 °C with air used for gassing at a rate of 1.5 L kg_{(total weight)}⁻¹ min⁻¹ and agitation with 2 Rushton-impellers set to 1000 rpm, ensuring dissolved oxygen levels above 25 % of the dissolved amount in the fermentation medium before the inoculation of the fermenter with cells of the filamentous fungus. The pH was kept at 5 by automated addition of ammonia (12.5 wt.-% aqueous solution) and H₂SO₄ (4M).

The fungal strain used was a *Trichoderma reesei* RUT-C30 derived strain in which the genes for all cellulases had been disrupted and which had been genetically modified to produce glucoamylase (EC number 3.2.1.3).

After 24 hours and the consumption of the initially added glucose, a solution (feeding-solution) containing glucose (3.3 wt.-%) and ammonium sulfate (0.21 wt.-%) was continuously pumped into the bioreactor over the course of 120 hours. The feeding-solution comprised 12 wt.-% of the total weight. After 24, 48, 72 and 98 hours 10 FPU kg_{(total weight)}⁻¹ cellulase was added to the fermenter. The addition of the cellulase was omitted in reference fermentations, i.e. the state of the art process.

Comparison of the "final biomass concentration" (dry weight of the cells present by the end of the fermentation), the "final volumetric enzymatic activity" and the "total amount of ammonia" (volume of base (12.5 wt-% ammonia) pumped into the reactor by the end of the fermentation over the course of the entire process to keep the pH at a pre-set value) used for pH correction can be seen in figures 1 to 4. The comparison shows significant increase of the final protein concentration and the final volumetric enzymatic activity for the inventive process as compared to the state of the art process. The final biomass concentration as well as the total amount of ammonia used for pH correction are both significantly reduced in the inventive process as compared to the state of the art process.

## Claims

1. Method for the production of proteins comprising the following steps:
(a) providing a fermentation medium comprising from 0.01 to 30 wt.-% cellulose;
(b) degermination of the fermentation medium;
(c) adding at least one filamentous fungus cell to the medium;
(d) adding from 0.01 to 35 wt.-% of glucose and/or saccharose over a period of at least 1 hour;
(e) adding from 0.1 to 10000 FPU cellulase per kg fermentation medium to the medium
wherein the at least one filamentous fungus cell is a recombinant cell and
wherein the at least one filamentous fungus cell is a cell wherein the expression level of one or more cellulases has been reduced and/or one or more cellulase encoding genes have been knocked out.

2. Method according to claim 1, wherein at least steps (c) to (e) are carried out concurrently or in a consecutive fashion.

3. Method according to any of the preceding claims, wherein step (d) is carried out in a continuous fashion.

4. Method according to any of the preceding claims, wherein the 0.1 to 10000 FPU cellulase per kg fermentation medium are added in from 1 to 500 portions or in a continuous fashion.

5. Method according to any of the preceding claims, wherein the pH is kept at a constant level by adding base or acid.

6. Method according to any of the preceding claims, wherein the filamentous fungus cell is selected from the group consisting of Acremonium, Aspergillus, Chaetomium, Fusarium, Humicola, Irpex, Magnaporte, Mucor, Myceliophthora, Neurospora, Penicillium, Rhizomucor, Rhizopus, Talaromyces, Trametes and Trichoderma species.

7. Method according to any of the preceding claims, wherein the protein is selected from the group of enzymes, consisting of esterases, hydrolases, lyases and oxidases.

8. Method according to any of the preceding claims, wherein at least steps (c) and (d) are carried out at a temperature of from 10 to 45 °C and/or at a pH of from 2.5 to 9.5 and/or for a time period of from 1 minute to 14 days and/or with a gassing rate of from 0.2 to 3 vvm and/or at a pressure of from 1 bar to 3 bar and/or with a a mechanical power input of from 0.01 to 20 kW/m3.

## Patentansprüche

1. Verfahren zur Herstellung von Proteinen, welches die folgenden Schritte umfasst:
(a) die Bereitstellung eines 0,01 bis 30 Gew.-% Cellulose umfassenden Fermentationsmediums,
(b) die Entkeimung des Fermentationsmediums,
(c) die Zugabe mindestens einer filamentösen Pilzzelle zum Medium,
(d) die Zugabe von 0,01 bis 35 Gew.-% Glucose und/oder Saccharose über eine Zeitspanne von mindestens 1 Stunde,
(e) die Zugabe von 0,1 bis 10 000 FPU Cellulase pro kg Fermentationsmedium zum Medium,
wobei es sich bei der mindestens einen filamentösen Pilzzelle um eine rekombinante Zelle handelt und
wobei es sich bei der mindestens einen filamentösen Pilzzelle um eine Zelle handelt, bei der das Expressionsniveau einer oder mehrerer Cellulasen reduziert und/oder ein oder mehrere für Cellulase codierende Gene durch Knockout abgeschaltet wurden.

2. Verfahren nach Anspruch 1, wobei mindestens die Schritte (c) bis (e) nebeneinander oder in aufeinanderfolgender Weise durchgeführt werden.

3. Verfahren nach einem der vorhergehenden Ansprüche, wobei Schritt (d) kontinuierlich durchgeführt wird.

4. Verfahren nach einem der vorhergehenden Ansprüche, wobei die 0,1 bis 10 000 FPU Cellulase pro kg Fermentationsmedium in 1 bis 500 Portionen oder kontinuierlich zugesetzt werden.

5. Verfahren nach einem der vorhergehenden Ansprüche, wobei der pH-Wert durch Zugabe von Base oder Säure konstant gehalten wird.

6. Verfahren nach einem der vorhergehenden Ansprüche, wobei die filamentöse Pilzzelle aus der aus Acremonium-, Aspergillus-, Chaetomium-, Fusarium-, Humicola-, Irpex-, Magnaporte-, Mucor-, Myceliophthora-, Neurospora-, Penicillium-, Rhizomucor-, Rhizopus-, Talaromyces-, Trametes- und Trichoderma-Arten bestehenden Gruppe ausgewählt ist.

7. Verfahren nach einem der vorhergehenden Ansprüche, wobei das Protein aus der aus Esterasen, Hydrolasen, Lyasen und Oxidasen bestehenden Gruppe von Enzymen ausgewählt ist.

8. Verfahren nach einem der vorhergehenden Ansprüche, wobei mindestens die Schritte (c) und (d) bei einer Temperatur von 10 bis 45°C und/oder bei einem pH-Wert von 2,5 bis 9,5 und/oder über eine Zeitspanne von 1 Minute bis 14 Tage und/oder mit einer Begasungsrate von 0,2 bis 3 vvm und/oder bei einem Druck von 1 bar bis 3 bar und/oder mit einem mechanischen Leistungseintrag von 0,01 bis 20 kW/m3 durchgeführt werden.

## Revendications

1. Procédé pour la production de protéines comprenant les étapes suivantes :
(a) mise à disposition d'un milieu de fermentation comprenant de 0,01 à 30 % en poids de cellulose ;
(b) dégermination du milieu de fermentation ;
(c) ajout d'au moins une cellule de champignon filamenteux au milieu ;
(d) ajout de 0,01 à 35 % en poids de glucose et/ou de saccharose sur une période d'au moins 1 heure ;
(e) ajout de 0,1 à 10 000 FPU de cellulase par kg de milieu de fermentation au milieu
l'au moins une cellule de champignon filamenteux étant une cellule recombinante et l'au moins une cellule de champignon filamenteux étant une cellule dans laquelle le niveau d'expression d'une ou plusieurs cellulases a été réduit et/ou un ou plusieurs gènes codant pour la cellulase ont été inactivés.

2. Procédé selon la revendication 1, au moins les étapes (c) à (e) étant mises en œuvre concurremment ou d'une manière consécutive.

3. Procédé selon l'une quelconque des revendications précédentes, l'étape (d) étant mise en œuvre d'une manière continue.

4. Procédé selon l'une quelconque des revendications précédentes, les 0,1 à 10 000 FPU de cellulase par kg de milieu de fermentation étant ajoutés à raison de 1 à 500 portions ou d'une manière continue.

5. Procédé selon l'une quelconque des revendications précédentes, le pH étant maintenu à un niveau constant par ajout de base ou d'acide.

6. Procédé selon l'une quelconque des revendications précédentes, la cellule de champignon filamenteux étant choisie dans le groupe constitué par les espèces Acremonium, Aspergillus, Chaetomium, Fusarium, Humicola, Irpex, Magnaporte, Mucor, Myceliophthora, Neurospora, Pénicillium, Rhizomucor, Rhizopus, Talaromyces, Trametes et Trichoderma.

7. Procédé selon l'une quelconque des revendications précédentes, la protéine étant choisie dans le groupe des enzymes, constitué par les estérases, les hydrolases, les lyases et les oxydases.

8. Procédé selon l'une quelconque des revendications précédentes, au moins les étapes (c) et (d) étant mises en œuvre à une température de 10 à 45 °C et/ou à un pH de 2,5 à 9,5 et/ou pendant une période de temps de 1 minute à 14 jours et/ou avec une vitesse de gazage de 0,2 à 3 vvm et/ou à une pression de 1 bar à 3 bars et/ou avec une entrée de puissance mécanique de 0,01 à 20 kW/m³.
